# EUROPEAN PATENT SPECIFICATION

(11) **EP 3 052 978 B1**
(45) Date of publication and mention of the grant of the patent: **28.11.2018**
(21) Application number: 14777855.9
(22) Date of filing: 10.09.2014
(51) Int. Cl.: G02B 6/36, G02B 6/24

(54) **LAUNCH FIXTURE FOR OPTICAL SHAPE SENSING**
STARTARMATUR FÜR OPTISCHE FORMMESSUNG
ACCESSOIRE DE LANCEMENT POUR UNE DÉTECTION DE FORME OPTIQUE

(30) Priority: 30.09.2013 US 201361884178 P
(43) Date of publication of application: 10.08.2016
(73) Proprietor: Koninklijke Philips N.V., 5656 AE Eindhoven (NL)
(72) Inventor: NOONAN, David Paul, NL-5656 AE Eindhoven (NL); FLEXMAN, Molly Lara, NL-5656 AE Eindhoven (NL); RAMACHANDRAN, Bharat, NL-5656 AE Eindhoven (NL); LEISTIKOW, Merel Danielle, NL-5656 AE Eindhoven (NL)
(74) Representative: Gravendeel, Cornelis
(86) International application number: PCT/IB2014/064361
(87) International publication number: WO 2015/044814

(56) References cited:
- EP-A1- 0 051 915
- WO-A1-2011/100124
- WO-A1-2013/061212
- US-A- 4 325 607
- US-A1- 2004 037 509
- US-A1- 2012 224 823

## Description

### BACKGROUND:

### Technical Field

This disclosure relates to medical instruments and more particularly to shape sensing optical fibers in medical applications.

### Description of the Related Art

Optical shape sensing (OSS) is a technology that permits accurate three-dimensional (3D) reconstruction of a shape of a fiber along its entire length. Integrating the fiber into an OSS enabled instrument requires coupling the fiber to the instrument such that any movement of the device can be reconstructed with respect to a fixed frame of reference. Having a fixed or static launch region is needed since the fixed region provides a search template for a reconstruction algorithm to perform correlation and to match a reference to sample data. The reconstruction initializes after this fixed region. The fixed region also defines the frame of reference (0,0,0) for OSS and, in essence, the coordinate system in the shape sensing space.

Various devices are disclosed in U.S. Publication No. 2004/0375 09, which discloses a launch fixture according to the preamble of claim 1; WO Publication No. 2013/061212; EP Publication No. 0051915; and U.S. Patent No. 4,325,607.

### SUMMARY

The invention is defined in the appended set of claims. In accordance with the present principles, a launch fixture for optical shape sensing (OSS) includes a first fixation device configured to receive and secure an optical fiber. A storage area is configured to receive and maintain the optical fiber within specified dimensions. A second fixation device is configured to receive and secure a flexible OSS enabled instrument. A launch region is configured to receive and maintain the optical fiber in aligning and coupling to a launch fixture base, which is configured to secure the launch fixture.

An optical shape sensing (OSS) system includes a launch fixture base configured to be connected to a support structure, and a launch fixture configured to be secured on the launch fixture base by at least one feature for aligning and coupling the launch fixture base to the launch fixture. The launch fixture includes a first fixation device configured to receive and secure an optical fiber; a fiber storage area configured to receive and maintain the optical fiber within specified dimensions; a second fixation device configured to receive and secure a flexible OSS enabled instrument; and a launch region configured to receive and maintain the optical fiber in a known geometric configuration before entering the second fixation device.

A method for optical shape sensing (OSS) includes providing (502) a launch fixture having a first fixation device configured to receive and secure an optical fiber, a fiber storage area configured to receive and maintain the optical fiber within specified dimensions, a second fixation device configured to receive and secure a flexible OSS enabled instrument, a launch region configured to receive and maintain the optical fiber in a known geometric configuration before entering the second fixation device, and at least one feature for aligning and coupling to a launch fixture base, which is configured to secure the launch fixture; and sensing a shape of the optical fiber.

These and other objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of preferred embodiments with reference to the following figures wherein:
FIG. 1 is a block/flow diagram showing a shape sensing system which employs a launch fixture with controlled fiber positioning in accordance with one embodiment;
FIG. 2 is a perspective view of a launch fixture with controlled fiber positioning in accordance with one embodiment;
FIG. 3 is a perspective view of a launch fixture base configured to secure one or more launch fixtures in accordance with one embodiment;
FIG. 4 is a perspective view showing a plurality of stacked launch fixtures with controlled fiber positioning in accordance with one embodiment;
FIG. 5 is a side view of a launch fixture base rack for storing a plurality of launch fixtures in accordance with one embodiment;
FIG. 6 is a perspective view of a launch fixture for handling a plurality of shape sensing devices with controlled fiber positioning in accordance with another embodiment;
FIG. 7 is a perspective view of a modular launch fixture with attachable/detachable modules in accordance with one embodiment;
FIG. 8A is a side view showing a sterile boundary between a launch fixture and a launch fixture base in accordance with one embodiment;
FIG. 8B is a top view showing an open launch fixture with a partitioned sterile boundary between distal and proximal portions of the launch fixture in accordance with one embodiment;
FIG. 9 is a top view of a rail for conveying a launch fixture base in accordance with one embodiment; and
FIG. 10 is a flow diagram showing a method for optical shape sensing in accordance with an illustrative embodiment.

### DETAILED DESCRIPTION OF EMBODIMENTS

In accordance with the present principles, a launch fixture and launch fixture base design provide for rapid attachment and detachment of one or more optical shape sensing (OSS) enabled flexible instrument(s) to a fixed reference in an operating theatre. The fixture can also be disposed in a transition zone between sterile and non-sterile regions in an operating theatre, allowing for the rapid exchange of OSS-enabled devices by personnel in the non-sterile region without breaking the sterile barrier.

In one embodiment, the launch fixture ensures that the geometric relationship between adjacent instrument frames of reference and the launch fixture base frame of reference are known, so that each instrument is automatically registered to the launch fixture base once connected. Given that the launch fixture base is registered to a patient or imaging frame of reference prior to a beginning of the procedure, this architecture permits each instrument to be rapidly deployed in a co-registered frame of reference. In addition, the overall footprint of the instruments in the operating theatre can be minimized through the use of vertical, horizontal, or angled stacking.

During clinical use, it is likely that multiple shape sensing enabled instruments will be deployed and exchanged during a given procedure. In one system architecture, each instrument could be attached to the operating table using a unique launch fixture base. However, as the number of instruments employed increases, a number of components attached to the table would increase accordingly. This would hinder clinician movement around the table and result in a cluttered operating field. In addition, since the OSS fiber within each instrument will reconstruct the shape of the instrument with respect to a frame of reference located within that instruments' launch fixture, each instrument would need to be individually registered to the patient/imaging system frame of reference.

In accordance with the present principles, the footprint of the instrument launch fixtures within the operating theatre is minimized, as well as the time spent registering devices. The launch fixture and launch fixture base permit rapid exchange of devices while maintaining a defined geometric relationship between frames of reference so that re-registration is not required. This fixed frame of reference is included within a launch fixture, which is coupled to a proximal end of the flexible instrument and includes design features which allow the fiber to be safely and securely coupled to the instrument. By attaching this launch fixture rigidly to the launch fixture base, which may itself be rigidly connected to the operating table, needed transformations between an instrument frame of reference and patient/imaging frames of reference can be computed. Thus, the reconstructed shape of the instrument can be overlaid on pre- and intra-operative images and used for navigation purposes.

It should be understood that the present invention will be described in terms of medical instruments; however, the teachings of the present invention are much broader and are applicable to any fiber optic shape sensed instruments. In some embodiments, the present principles are employed in tracking or analyzing complex biological or mechanical systems. In particular, the present principles are applicable to internal tracking procedures of biological systems, procedures in all areas of the body such as the lungs, gastro-intestinal tract, excretory organs, blood vessels, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray™ and DVD

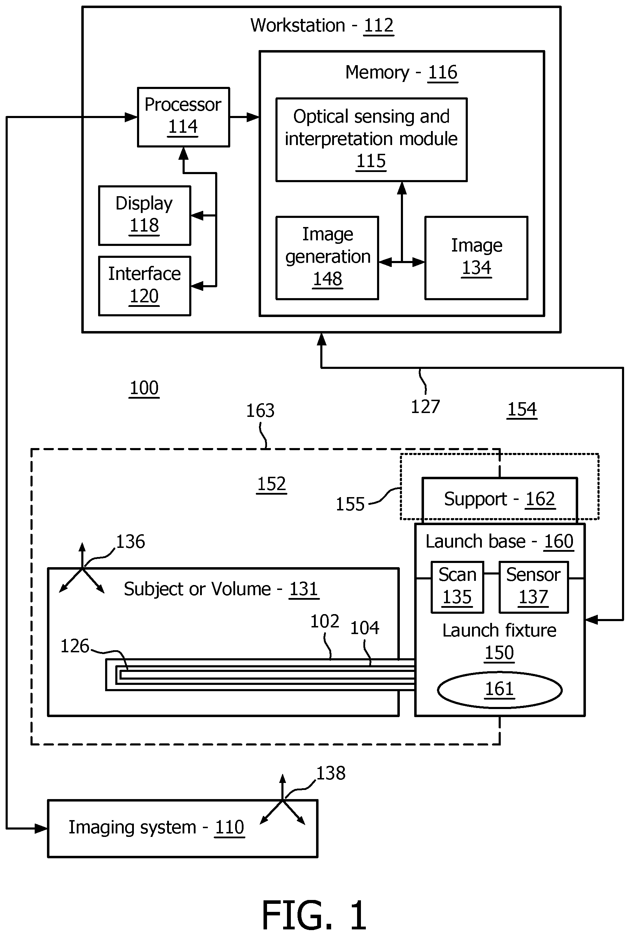

Referring now to the drawings in which like numerals represent the same or similar elements and initially to FIG. 1, a system 100 for optical shape sensing is illustratively shown in accordance with present embodiments. System 100 may include a workstation or console 112 from which a procedure is supervised and/or managed. Workstation 112 preferably includes one or more processors 114 and memory 116 for storing programs and applications. Memory 116 may store an optical sensing module 115 configured to interpret optical feedback signals from a shape sensing device or system 104. Optical sensing module 115 is configured to use the optical signal feedback (and any other feedback, e.g., electromagnetic (EM) tracking) to reconstruct deformations, deflections and other changes associated with a medical device or instrument 102 and/or its surrounding region. The medical device 102 may include a catheter, a guidewire, a probe, an endoscope, a robot, an electrode, a filter device, a balloon device, or other medical component, etc.

The shape sensing system 104 on device 102 includes one or more optical fibers 126 which are coupled to the device 102 in a set pattern or patterns. The optical fibers 126 connect to the workstation 112 through cabling 127. The cabling 127 may include fiber optics, electrical connections, other instrumentation, etc., as needed.

Shape sensing system 104 with fiber optics may be based on fiber optic Bragg grating sensors. A fiber optic Bragg grating (FBG) is a short segment of optical fiber that reflects particular wavelengths of light and transmits all others. This is achieved by adding a periodic variation of the refractive index in the fiber core, which generates a wavelength-specific dielectric mirror. A fiber Bragg grating can therefore be used as an inline optical filter to block certain wavelengths, or as a wavelength-specific reflector.

A fundamental principle behind the operation of a fiber Bragg grating is Fresnel reflection at each of the interfaces where the refractive index is changing. For some wavelengths, the reflected light of the various periods is in phase so that constructive interference exists for reflection and, consequently, destructive interference for transmission. The Bragg wavelength is sensitive to strain as well as to temperature. This means that Bragg gratings can be used as sensing elements in fiber optical sensors. In an FBG sensor, the measurand (e.g., strain) causes a shift in the Bragg wavelength.

One advantage of this technique is that various sensor elements can be distributed over the length of a fiber. Incorporating three or more cores with various sensors (gauges) along the length of a fiber that is embedded in a structure permits a three dimensional form of such a structure to be precisely determined, typically with better than 1 mm accuracy. Along the length of the fiber, at various positions, a multitude of FBG sensors can be located (e.g., 3 or more fiber sensing cores). From the strain measurement of each FBG, the curvature of the structure can be inferred at that position. From the multitude of measured positions, the total three-dimensional form is determined.

As an alternative to fiber-optic Bragg gratings, the inherent backscatter in conventional optical fiber can be exploited. One such approach is to use Rayleigh scatter in standard single-mode communications fiber. Rayleigh scatter occurs as a result of random fluctuations of the index of refraction in the fiber core. These random fluctuations can be modeled as a Bragg grating with a random variation of amplitude and phase along the grating length. By using this effect in three or more cores running within a single length of multi-core fiber, the 3D shape and dynamics of the surface of interest can be followed.

In one embodiment, workstation 112 includes an image generation module 148 configured to receive feedback from the shape sensing device 104 and display position/shape for the shape sensing device 104 within a volume 131. An image 134 of the shape sensing device 104 within the space or volume 131 can be displayed on a display device 118. Workstation 112 includes the display 118 for viewing internal images of a subject (patient) or volume 131 and may include the image 134 as an overlay or other rendering of shapes of the shape sensing device 104. Display 118 may also permit a user to interact with the workstation 112 and its components and functions, or any other element within the system 100. This is further facilitated by an interface 120 which may include a keyboard, mouse, a joystick, a haptic device, or any other peripheral or control to permit user feedback from and interaction with the workstation 112.

A launch fixture 150 includes mechanical features 152 configured to ensure that the fiber 126 or the shape sensing device 104 can be coupled to the elongate instrument 102 such that the fiber measures changes in shape of the instrument with respect to a fixed frame of reference. Each OSS enabled instrument 102 employs a launch fixture (150) to define a fixed frame of reference which may be registered to a patient frame of reference 136, or imaging frame of reference 138, or both.

A launch fixture base 160 is rigidly attached to a fixed feature or support structure 162 in the operating theatre (e.g., an operating table or similar structure) which allows for multiple launch fixtures 150 (and hence multiple OSS enabled instruments 102) to be attached and co-registered to the patient frame of reference 136, or imaging frame of reference 138, or both. In another embodiment, there is more than one OSS enabled instrument 104 within a single fixture 150. The launch and coordinate systems of these fibers are known with respect to each other as well as the patient and imaging frames of reference 136, 138, thus allowing faster use when these devices are used in conjunction, such as, e.g., a guidewire and a catheter being used together.

In another embodiment, the launch base 160 and/or the launch fixture 150 may include features to monitor the use of the launch fixture 150 and devices 102. For example, a scan mechanism 135 may be incorporated into the base 160 or fixture 150 (e.g., a radiofrequency scanner) that automatically detects which OSS enabled instrument 102 is being used. Mechanism 135 may monitor a number of times a same device is clipped in (to monitor or prevent) more than one-time use of the device 102. The mechanism 135 may include an indicator (e.g., a light, counter or color strip) that indicates an end of life (after say 1000 devices have been mounted) of the launch base 150 and alert the service engineer for maintenance. Other configurations for mechanism 135 are also contemplated.

In one embodiment, a position of the launch fixture base 160 (and/or launch fixture 150) with respect to an imaging system 110 is tracked using an appropriate sensor 137 (position encoder, magnetic tracking, etc.). This provides easy registration to the imaging system 110.

Another feature of the launch fixture 150 is that it may lay in a zone separating a sterile region 152 (inside dashed box 153) and a non-sterile region 154 in a surgical room. A sterile barrier 153 may be provided such that a proximal portion of device 150 (in region 154) is non-sterile and a distal portion of the device 150 (in region 154) is in the sterile region, which includes a mechanism (a barrier or sealed partition within the device 150) for not contaminating the sterile region 152 in an operating room. In one possible workflow, by allowing the non-sterile personnel such as a nurse to perform the proximal connections to a laser and clip on the launch fixture 150 to the launch base 160 and handing a protected OSS-enabled instrument 104 to the sterile personnel, the launch fixture 150 allows use of OSS-enabled devices 104 without breaking the sterile barrier. In another possible workflow, the launch base 160 is also sterile and connects to the support 162 over a sterile drape 155. This combination also allows for the launch fixture 150 to be employed to enable the use of OSS instruments 102 without breaking the sterile barrier 153.

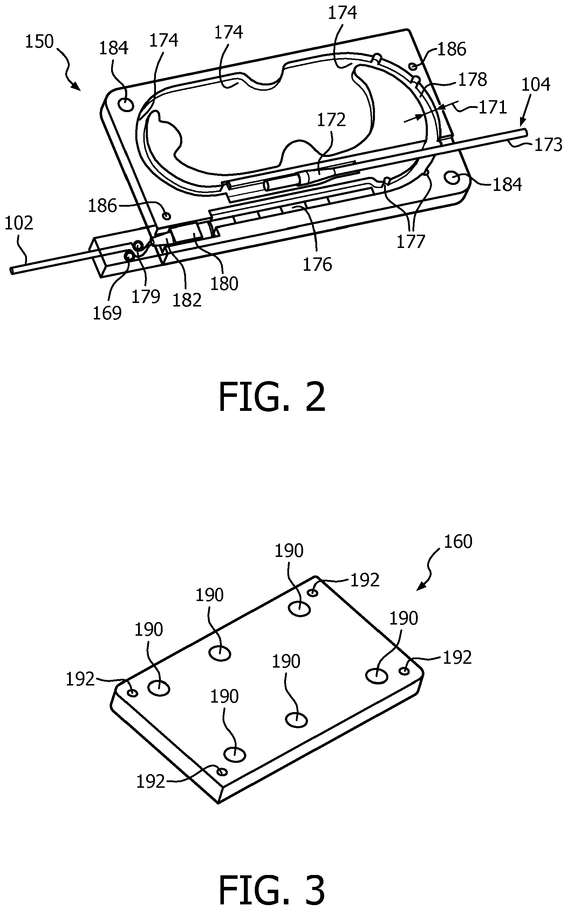

Referring to FIG. 2, a launch fixture 150 suitable for coupling an OSS fiber device 104 to a flexible instrument 102 in a controlled manner is illustratively shown. The launch fixture 150 includes a fixation point or device 172 to clamp the optical fiber 104 and its protective tubing, housing, or similar structure 173 in place into the fixture 150. A defined path 174 is formed to permit excess fiber to be included within the fixture 150 while not exceeding a specified minimum bend radius. This path 174 may also include features which allow for automatic alignment of the polarization of the OSS laser system to be performed while the instrument is connected to the OSS console. When a fiber undergoes a tight bend the index of refraction experienced by the light through that bend will vary depending on the orientation of the light. To mitigate these birefringence effects, optical shape sensing measurements are commonly performed with multiple light polarizations. One technique to optimally select (or 'align') those polarization states is to use the measured optical response through a known feature, such as a bend. An example of such a feature is a defined path 174 with sufficient curvature to induce birefringence effects in the fiber. These birefringence effects provide unique features in the measured optical signal that can be used for automatic alignment of the system polarization. Ideally, this curvature will have a tight radius to amplify the birefringence effects and thus allow for a more unique feature upon which to perform alignment. This alignment feature can exist prior to a launch region 176 within the launch fixture 150.

The launch region 176 permits the OSS fiber device 104 to be physically attached to the fixture 150 in a defined manner (e.g., straight or with a known geometry). The path prior to the launch region 176 may have features 177 which ensure the fiber enters the launch region 176 in a controlled manner (e.g., pegs, radiused features, etc.). A path 178 (which may be included in path 174) acts as a buffer, or service loop, to allow for curvature induced path length changes to be accommodated by the fiber repositioning within the fixture 150 while not exceeding a specified minimum bend radius. Such a service loop may take several different forms, e.g., the service buffer loop of path 178 may include a 90 degree, 360 degree bend, etc. instead of or in addition to the 180 degree bend depicted in FIG. 2. A gap distance 171 of the path 178 provides slack for manipulating the fiber but protects the fiber from exceeding the minimum bend radius.

A fixation point or device 180 clamps the flexible instrument 102 to the fixture 150 and surface curvatures/fillets 182 ensure that the transition between the fixture 150 and the flexible instrument 102 occurs without negatively effecting the strain measurement of the OSS fiber 104. Mechanical design features 184 allow the fixture 150 to be reproducibly connected to the launch fixture base 160 (FIG. 1), or other launch fixtures in a known geometric manner. Screws, magnets, snap-fits, clips, pegs, or similar mechanisms can be employed to achieve this. Feature or features 184 with known geometry, curvature or shape, may be employed for registering the device 102 to other OSS-enabled devices, the patient and the imaging frame of reference 136, 138. The feature(s) 184 may be employed for the dual purpose of attaching the fixture 150 to the launch base 160 and attaching fixtures 150 to each other. The feature(s) 184 may be directional shaped (e.g., square, triangle, etc.) to ensure that the base 160 and the fixtures 150 are properly aligned relative to one another.

The launch fixture 150 is depicted in an open configuration in FIG. 2. A lid (not shown) can be employed to cover the open launch fixture 150 or another launch fixture (not shown) may nest or be coupled to the launch fixture 150. The lid or additional launch fixture (150) may be screwed or otherwise connected into place to both clamp the components into place (on the base 160) and to protect the fiber from the environment by employing features 186. Feature 186 may include similar mechanism and described for features 184.

An additional feature of the launch fixture 150 includes a defined path 179 for the fiber distal from the launch region 176 which can be used to correct for any rotation of the launch region during use. In practice, the fiber is clamped in place at the launch region 176; however, in some circumstances the fiber can 'rotate' within the clamp and thus any registrations can become inaccurate. By having a known shape distal from the launch region, this rotation can be corrected for. The defined path 179 may include a curved path in two or three dimensions, e.g., a semi-circle, compound curves (e.g., sinusoid), an arc, a coiled shape, etc. The defined path 179 may be included distally to the launch region 176 within the launch fixture 150 or may be included as a detachable module 169 that connects to the launch fixture (as depicted in FIG. 2).

Referring to FIG. 3, a launch fixture base 160 is shown in accordance with one illustrative embodiment. The launch fixture base 160 includes features 190, such as holes, pegs, detents, protrusions, etc. configured to rigidly attach to a fixed frame of reference within the operating theatre (e.g., the operating table or the like). The launch fixture base 160 includes the ability to be reproducibly connected to one or more launch fixtures 150 with a known geometric relationship. This may include features 192, such as holes, pegs, detents, protrusions, etc. configured to permit easier connection and fixation of the launch fixture(s) 150 or a lid on the launch fixture(s) 150.

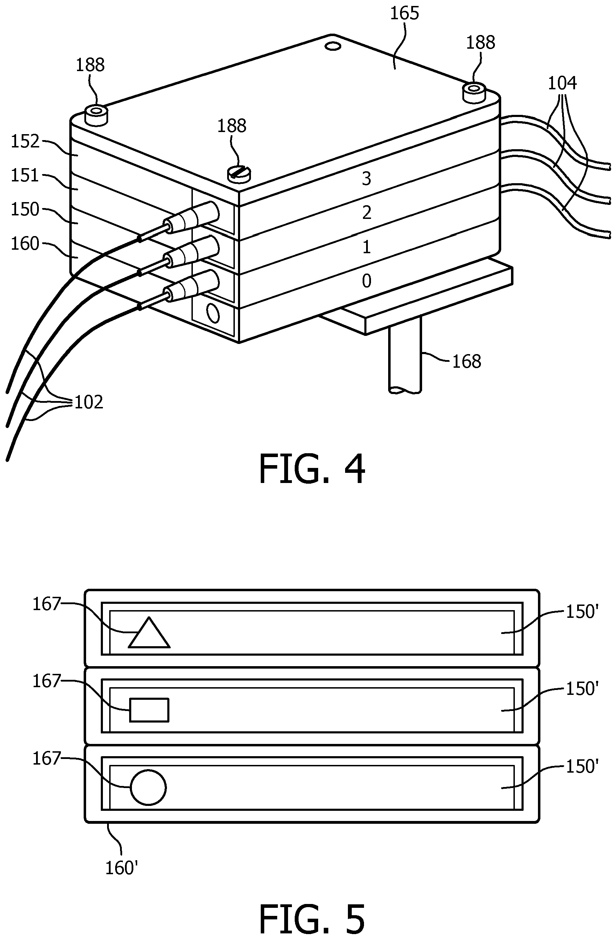

Referring to FIG. 4, three launch fixtures 150, 151 and 153 are connected to a launch fixture base 160 which is, in turn, connected to an operating table (not shown) via steel rods and support structure 163 and appropriate clamps, etc. In this architecture, the transformations between the frames of reference of the individual launch fixtures (and hence the flexible instruments 102 connected to the launch fixtures 150, 151, 153) and the launch fixture base 160 are known. Assuming a transformation between the launch fixture base 160 and the patient or imaging system frame of reference is known, then different launch fixtures 150, 151, 153 can be connected to the launch fixture base 160 and used for imaged guided navigation without requiring a re-registration step. The known geometric relationship between each launch fixture 150, 151, 153 and the launch fixture base 160 can be used to minimize time spent registering each device to the patient/imaging system frame of reference.

In one embodiment, launch fixtures 150, 151, 153 can be rigidly attached to the launch fixture base 160 using threaded bolts 188. A lid 165 may be employed as well and secured together with fixtures150, 151, 153. For example, additional launch fixtures 151, 153 can be attached to the initial launch fixture 150 with lid 165 using longer bolts. In other embodiments, magnets, clips, snap fits or similar rapid attachment components may be employed instead of bolts to attach and detach the launch fixture(s) from each other and the launch fixture base 160. Quick connect or magnetic attachment mechanisms (e.g., snap together arrangements, clamps, clasps, tongue and groove, etc.) may be employed to make quick and secure but releasable connections between the base 160 and fixtures 150, 151, 153 and between fixtures 150, 151, 153. The geometric relationship between adjacent launch fixtures 150, 151, 153 (and hence their instrument frames of reference) and the launch fixture base 160 can be used to ensure that re-registration of multiple devices is not required as devices are exchanged during a procedure. In addition, by locating the fixtures 150, 151, 153 and base 160 at a single location clutter is reduced and an organized and efficient operating theater is maintained.

Referring to FIG. 5, in another embodiment, launch fixtures 150' couple to a launch fixture base 160' independently (e.g., not stacked on each other). Examples of such an embodiment include a rack-style base or similar architecture where the fixtures can be stacked horizontally, vertically or angled with respect to an operating table or other reference structure.

In one embodiment, connection features 163 may include different shapes for receiving different types of OSS enabled devices 102. For example, a square, triangle, circle, etc. feature 163 may be employed to limit the type of OSS enabled device 102 connected to the launch fixture 150'. For example, a guide wire may include a square connector, a catheter may include a round connector and an endoscope may include a triangular connector.

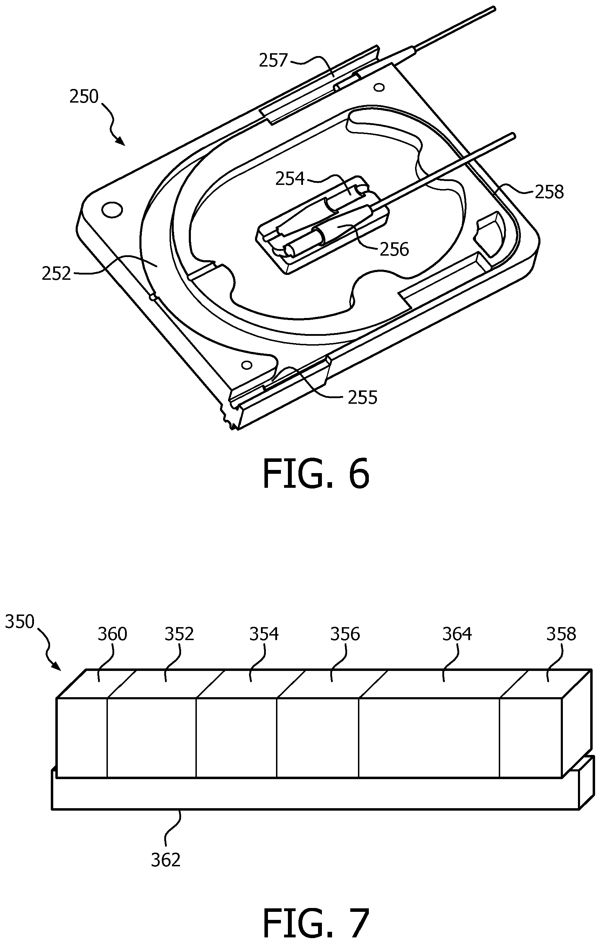

Referring to FIG. 6, in another embodiment, similar to that launch fixture 150, a launch fixture 250 is shown capable of coupling two OSS fibers 104 to two flexible instruments 102 within a single fixture 250. An entrance point 254 for one fiber and an exit point 255 for one flexible instrument 102 are depicted. An entrance point 256 for one fiber and an exit point 257 for another flexible instrument 102 are also depicted. In this embodiment, if two instruments 102 are employed within the single fixture 250, one instrument 102 may use a buffer, or service loop 252 and, the other instrument 102 may not employ the service loop 252. However, a service loop may be provided for both instruments in other embodiments. Other embodiments may employ a single fixture with a greater number of flexible instruments. A fiber path 258 may be employed for automatic alignment of light source polarization states, as described above.

Referring to FIG. 7, a modular launch fixture 350 is shown in accordance with one embodiment. The fixture 350 illustratively includes selected elements or features needed to couple an OSS fiber to a flexible instrument in a controlled manner. The launch fixture 350 in this particular embodiment adopts a modular approach to attaching each of a plurality of elements described above to a rail system or launch fixture base 362. This rail 362 can be attached to an operating table and then registered to the patient and/or imaging system frame of reference.

The launch fixture 350 illustratively includes a launch region module 352, a buffer or service loop module 354, an excess fiber path module 356, a fiber boot clamp module 358, and a clamp module 360 for the flexible instrument 102. Other modules 364 may be included. The modules 352, 354, 356, 358, 362, 364 are individually separable from the launch fixture base 360 and adjacent modules. The modular embodiment of the launch fixture 350 may include multiple flexible instruments and/or stackable launch fixtures, each with one or more flexible instruments. The fibers in each module may include optical connectors so that modules can be easily changed out as needed.

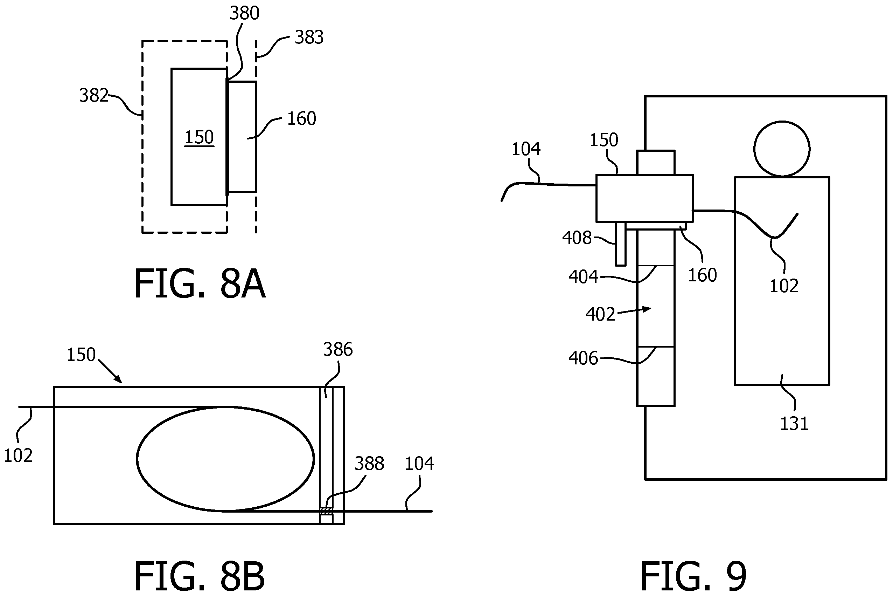

Referring to FIG. 8A, a protective layer 380 (e.g., a plastic film, sterile drape or the like) is disposed between the launch base 160 and the launch fixture 150, allowing a sterile device within a protected plastic 382 to clip on the non-sterile launch-base 160 while still having the packaging that maintains sterility. Others arrangements are also contemplated.

In another embodiment, the launch fixture base 160 can be multi-use, sterilizable and connect over a sterile drape 383. The launch fixture 150 can be connected to the sterile launch fixture base 160 thus allowing the OSS device to be used within the sterile field.

Referring to FIG. 8B, a protective partition 386 (e.g., a stainless steel wall) with sealed orifices 388 is disposed in the launch fixture 150, allowing a sterile device on a distal side (with instrument 102) and a non-sterile device on a proximal side (with fiber 104). Others arrangements are also contemplated.

Referring to FIG. 9, in another embodiment, the launch fixture base 160 is coupled to a rail 402 or other structure such that the OSS enabled device 102 could be used for entering a body 131 at different locations, such as femoral access versus carotid access. The launch fixture base 160 could be repositioned at preset states or locations 404, 406 and lock into place by a clamp 408 or other securing device. The positions, orientations and transforms of the base 160 at these locations 404, 406 with respect to other locations would be known and thus, registering multiple OSS devices 102 would be straightforward and simple. In one embodiment, the launch fixture 150 and/or launch fixture base 160 may be robotically actuated and manipulated to different positions with or without the rail 402 (the rail 402 may also be robotically positioned). Translation and rotation of the rail 402, the launch fixture 150 and/or launch fixture base 160 may be provided robotically for medical procedures (e.g., endovascular, endoluminal and/or orthopedic applications, etc.) or other applications. In another embodiment, translation along the rail 402 may be performed manually, and the transformations to the patient or imaging coordinate systems, or both, can be calculated by measuring the position along the rail 402 using a position sensing device (potentiometer, optical tracker, etc.). If a robot is employed, the fixtures 150 and base 160 may be registered to the robotic coordinate system.

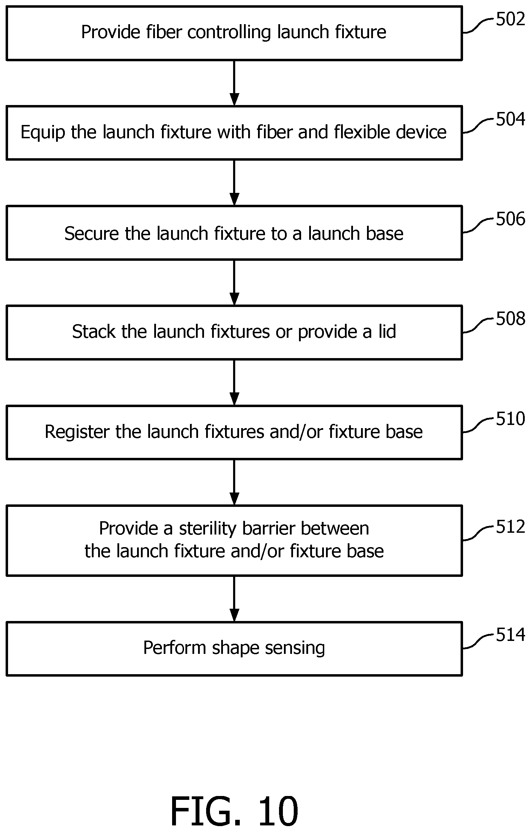

Referring to FIG. 10, a method for optical shape sensing (OSS) includes providing a launch fixture for handling optical fiber in a controlled manner, in block 502. The launch fixture includes a first fixation device configured to receive and secure an optical fiber, a fiber storage area configured to receive and maintain the optical fiber within specified dimensions, a second fixation device configured to receive and secure a flexible OSS enabled instrument, a launch region configured to receive and maintain the optical fiber in a known geometric configuration before entering the second fixation device, and at least one feature for aligning and coupling to a launch fixture base, which is configured to secure the launch fixture. The launch fixture may include other features as well, e.g., a defined path which can be used to correct for rotation of the fiber launch region (as described above).

In block 504, the launch fixture is equipped with at least one OSS fiber, which is initially secured in the first fixation device, and at least one OSS enabled device, which is secured in the second fixation device. The OSS fiber is stowed in buffer or loop area configured to maintain minimum dimensions requirements to the fiber and to provide some slack for accommodating operational movement of the fiber without exceeding the requirements. In block 506, the launch fixture is secured to a launch fixture base. The launch fixture base may be secured to an operating table, a rail system, robotic instrument, imaging system, etc. In one embodiment, the launch fixture base may be actuated to different position within an operating theater.

In block 508, other launch fixtures are stacked on the launch fixture and/or a lid is placed over the launch fixture. In block 510, the launch fixture or fixtures and/or fixture base are registered to at least one of a patient frame of reference and an imaging frame of reference, although other references may be employed. In block 512, a sterile barrier may be employed through the launch fixture(s), between the launch fixture and the launch fixture base or between modules of a modular launch fixture. In block 514, the flexible OSS enabled instrument is employed to sense a shape of the optical fiber.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

Having described preferred embodiments a launch fixture for optical shape sensing (which are intended to be illustrative and not limiting), it is noted that modifications and variations can be made by persons skilled in the art in light of the above teachings. It is therefore to be understood that changes may be made in the particular embodiments of the disclosure disclosed which are within the scope of the embodiments disclosed herein as outlined by the appended claims. Having thus described the details and particularity required by the patent laws, what is claimed and desired protected by Letters Patent is set forth in the appended claims.

## Claims

1. A launch fixture (150) for optical shape sensing (OSS), comprising:
a first fixation device (172) configured to receive and secure an optical fiber;
a fiber storage area (174) configured to receive and maintain the optical fiber within specified dimensions,
a second fixation device (180) configured to receive and secure a flexible OSS enabled instrument;
a launch region (176) configured to receive and maintain the optical fiber in a known geometric configuration before entering the second fixation device (180); and said launch fixture being **characterized in that** the fiber storage area (174) includes a service loop region (178) for storing fiber and accommodating repositioning of the optical fiber within permissible geometric constraints, and
at least one feature (184) for aligning and coupling to a launch fixture base, which is configured to secure the launch fixture.

2. The fixture as recited in claim 1, wherein the fixture includes an open side for loading the optical fiber therein and further comprising a lid (165) for closing the open side.

3. The fixture as recited in claim 1, further comprising a connecting feature (186) for aligning and coupling the launch fixture with one or more additional launch fixtures.

4. The fixture as recited in claim 1, further comprising a modular launch fixture (350) configured to permit functional portions of the launch fixture to be separately attachable and detachable to and from the launch fixture base.

5. The fixture as recited in claim 1, further comprising a geometric shape feature (174, 177, 178) configured to maintain a portion of the optical fiber in a known position to enable registration of the launch fixture with other instruments.

6. The fixture as recited in claim 1, wherein the fixture (250) is configured to accommodate a plurality of OSS enabled instruments.

7. The fixture as recited in claim 1, wherein the fiber storage area (174) includes a defined path for the fiber configured to optimize polarization alignment of a light source.

8. The fixture as recited in claim 1, further comprising a defined fiber path (179) disposed distally to the launch region to provide a check for fiber rotation in the launch region.

9. An optical shape sensing (OSS) system, comprising:
a launch fixture base (160) configured to be connected to a support structure; and
the launch fixture (150) as recited in claim 1 to be coupled to the launch fixture base.

10. The system as recited in claim 9, wherein the fixture includes an open side for loading the optical fiber therein and further comprising a lid (165) for closing the open side.

11. The system as recited in claim 9, further comprising a connecting feature (184) for aligning and coupling the launch fixture with one or more additional launch fixtures.

12. The system as recited in claim 9, further comprising a modular launch fixture (350) configured to permit functional portions of the launch fixture to be separately attachable and detachable to and from the launch fixture base.

13. A method for optical shape sensing (OSS), comprising:
providing (502) a launch fixture having a first fixation device configured to receive and secure an optical fiber, a fiber storage area configured to receive and maintain the optical fiber within specified dimensions, wherein the fiber storage area (174) includes a service loop region (178) for storing fiber and accommodating repositioning of the optical fiber within permissible geometric constraints, a second fixation device configured to receive and secure a flexible OSS enabled instrument, a launch region configured to receive and maintain the optical fiber in a known geometric configuration before entering the second fixation device, and at least one feature for aligning and coupling to a launch fixture base, which is configured to secure the launch fixture; and
sensing (514) a shape of the optical fiber.

14. The method as recited in claim 13, further comprising: registering (510) the launch fixture to at least one of a patient frame of reference and an imaging frame of reference.

## Patentansprüche

1. Optische Formmessung (OSS) für eine Startarmatur (150), umfassend:
eine erste Fixiervorrichtung (172), die konfiguriert ist, um eine optische Faser aufzunehmen und zu sichern;
einen Faserspeicherbereich (174), der konfiguriert ist, um die optische Faser innerhalb spezifizierter Dimensionen aufzunehmen und zu halten,
eine zweite Fixiervorrichtung (180), die konfiguriert ist, um ein flexibles OSS-fähiges Instrument aufzunehmen und zu sichern;
einen Startbereich (176), der konfiguriert ist, um die optische Faser in einer bekannten geometrischen Konfiguration aufzunehmen und zu halten, bevor sie in die zweite Fixiervorrichtung (180) eintritt;
und die Startarmatur **dadurch gekennzeichnet ist, dass** der Faserspeicherbereich (174) einen Serviceschleifenbereich (178) zum Speichern von Faser und zum Aufnehmen von Neupositionierung der optischen Faser innerhalb erlaubter geometrischer Beschränkungen beinhaltet, und
mindestens ein Merkmal (184) zum Ausrichten und Koppeln an eine Startarmatur-Basis, die zur Sicherung der Startarmatur konfiguriert ist.

2. Armatur nach Anspruch 1, wobei die Armatur eine offene Seite zum Einlegen der optischen Faser darin beinhaltet und weiter einen Deckel (165) zum Schließen der offenen Seite umfasst.

3. Armatur nach Anspruch 1, weiter umfassend ein Verbindungsmerkmal (186) zum Ausrichten und Koppeln der Startarmatur mit einer oder mehreren zusätzlichen Startarmaturen.

4. Armatur nach Anspruch 1, weiter umfassend eine modulare Startarmatur (350), die konfiguriert ist, um zu ermöglichen, dass Funktionsteile der Startarmatur separat an der Startarmatur-Basis anbringbar und von dieser abnehmbar sind.

5. Armatur nach Anspruch 1, weiter umfassend ein geometrisches Formmerkmal (174, 177, 178), das konfiguriert ist, um ein Teil der optischen Faser in einer bekannten Position zu halten, um eine Registrierung der Startarmatur mit anderen Instrumenten zu ermöglichen.

6. Armatur nach Anspruch 1, wobei die Armatur (250) konfiguriert ist, um eine Vielzahl von OSS-fähigen Instrumenten aufzunehmen.

7. Armatur nach Anspruch 1, wobei der Faserspeicherbereich (174) einen definierten Weg für die Faser beinhaltet, der konfiguriert ist, um die Polarisationsausrichtung einer Lichtquelle zu optimieren.

8. Armatur nach Anspruch 1, weiter umfassend einen definierten Faserweg (179), der distal zu dem Startbereich angeordnet ist, um eine Überprüfung der Faserrotation in dem Startbereich bereitzustellen.

9. Optisches Formmesssystem (OSS), umfassend:
eine Startarmatur-Basis (160), die konfiguriert ist, um mit einer Trägerstruktur verbunden zu werden; und
die Startarmatur (150) nach Anspruch 1, die an der Startarmatur-Basis gekoppelt ist.

10. System nach Anspruch 9, wobei die Armatur eine offene Seite zum Einlegen der optischen Faser darin beinhaltet und weiter einen Deckel (165) zum Schließen der offenen Seite umfasst.

11. System nach Anspruch 9, weiter umfassend ein Verbindungsmerkmal (184) zum Ausrichten und Koppeln der Startarmatur mit einer oder mehreren zusätzlichen Startarmaturen.

12. System nach Anspruch 9, weiter umfassend eine modulare Startarmatur (350), die konfiguriert ist, um zu ermöglichen, dass Funktionsteile der Startarmatur separat an der Startarmatur-Basis anbringbar und von dieser abnehmbar sind.

13. Verfahren zur optischen Formmessung (OSS), umfassend:
Bereitstellen (502) einer Startarmatur mit einer ersten Fixiervorrichtung, die konfiguriert ist, um eine optische Faser aufzunehmen und zu sichern, wobei ein Faserspeicherbereich konfiguriert ist, um die optische Faser innerhalb spezifizierter Dimensionen aufzunehmen und zu halten, wobei der Faserspeicherbereich (174) einen Dienstschleifenbereich (178) zum Speichern einer Faser und zum Aufnehmen einer Neupositionierung der optischen Faser innerhalb erlaubter geometrischer Beschränkungen beinhaltet, eine zweite Fixiervorrichtung, die konfiguriert ist, um ein flexibles OSS-fähiges Instrument aufzunehmen und zu sichern, ein Startbereich, der konfiguriert ist, um die optische Faser in einer bekannten geometrischen Konfiguration aufzunehmen und zu halten, bevor sie in die zweite Fixiervorrichtung eintritt, und mindestens ein Merkmal zum Ausrichten und Koppeln an einer Startarmatur-Basis, die zum Sichern der Startarmatur konfiguriert ist; und
Messung (514) einer Form der optischen Faser.

14. Verfahren nach Anspruch 13, weiter umfassend: Registrieren (510) der Startarmatur zu mindestens einem Patienten-Bezugssystem und einem Bildgebungs-Bezugssystem.

## Revendications

1. Accessoire de lancement (150) pour la détection de forme optique (OSS), comprenant :
un premier dispositif de fixation (172) configuré pour recevoir et fixer une fibre optique ;
une zone de stockage de fibre (174) configurée pour recevoir et maintenir la fibre optique dans les limites de dimensions spécifiées,
un second dispositif de fixation (180) configuré pour recevoir et fixer un instrument souple compatible OSS ;
une région de lancement (176) configurée pour recevoir et maintenir la fibre optique dans une configuration géométrique connue avant d'entrer le second dispositif de fixation (180) ;
et ledit accessoire de lancement étant **caractérisé en ce que** la zone de stockage de fibre (174) inclut une région de boucle de service (178) pour stocker la fibre et acceptant le repositionnement de la fibre optique dans des limites de contraintes géométriques admissibles, et
au moins une particularité (184) pour alignement et accouplement à une base d'accessoire de lancement, qui est configurée pour fixer l'accessoire de lancement.

2. Accessoire selon la revendication 1, dans lequel l'accessoire inclut un côté ouvert pour charger la fibre optique en son sein et comprenant en outre un couvercle (165) pour fermer le côté ouvert.

3. Accessoire selon la revendication 1, comprenant en outre une particularité de connexion (186) pour l'alignement et l'accouplement de l'accessoire de lancement à un ou plusieurs accessoires de lancement supplémentaires.

4. Accessoire selon la revendication 1, comprenant en outre un accessoire de lancement modulaire (350) configuré pour permettre à des portions fonctionnelles de l'accessoire de lancement de pouvoir être séparément attachées et détachées à/de la base d'accessoire de lancement.

5. Accessoire selon la revendication 1, comprenant en outre une particularité de forme géométrique (174, 177, 178) configurée pour maintenir une portion de la fibre optique dans une position connue pour permettre le calage de l'accessoire de lancement avec d'autres instruments.

6. Accessoire selon la revendication 1, dans lequel l'accessoire (250) est configuré pour accepter une pluralité d'instruments compatibles OSS.

7. Accessoire selon la revendication 1, dans lequel la zone de stockage de fibre (174) inclut un chemin défini pour la fibre, configuré pour optimiser l'alignement de polarisation d'une source de lumière.

8. Accessoire selon la revendication 1, comprenant en outre un chemin de fibre défini (179) disposé de façon distale par rapport à la région de lancement pour fournir un contrôle de la rotation de fibre dans la région de lancement.

9. Système de détection de forme optique (OSS), comprenant :
une base d'accessoire de lancement (160) configurée pour être raccordée à une structure de support ; et
l'accessoire de lancement (150) selon la revendication 1 à accoupler à la base d'accessoire de lancement.

10. Système selon la revendication 9, dans lequel l'accessoire inclut un côté ouvert pour charger la fibre optique en son sein et comprenant en outre un couvercle (165) pour fermer le côté ouvert.

11. Système selon la revendication 9, comprenant en outre une particularité de raccordement (184) pour l'alignement et l'accouplement de l'accessoire de lancement avec un ou plusieurs accessoires de lancement supplémentaires.

12. Système selon la revendication 9, comprenant en outre un accessoire de lancement modulaire (350) configuré pour permettre à des portions fonctionnelles de l'accessoire de lancement de pouvoir être séparément attachées et détachées à et de la base d'accessoire de lancement.

13. Procédé pour la détection de forme optique (OSS), comprenant :
la fourniture (502) d'un accessoire de lancement ayant un premier dispositif de fixation configuré pour recevoir et fixer une fibre optique, une zone de stockage de fibre configurée pour recevoir et maintenir la fibre optique dans des limites de dimensions spécifiées, dans lequel la zone de stockage de fibre (174) inclut une région de boucle de service (178) pour stocker la fibre et accepter le repositionnement de la fibre optique dans des limites de contraintes géométriques admissibles, un second dispositif de fixation configuré pour recevoir et fixer un instrument souple compatible OSS, une région de lancement configurée pour recevoir et maintenir la fibre optique dans une configuration géométrique connue avant d'entrer le second dispositif de fixation, et au moins une particularité pour l'alignement et l'accouplement à une base d'accessoire de lancement, qui est configurée pour fixer l'accessoire de lancement ; et
la détection (514) d'une forme de la fibre optique.

14. Procédé selon la revendication 13, comprenant en outre : le calage (510) de l'accessoire de lancement sur au moins l'un d'un cadre de référence de patient et d'un cadre de référence d'imagerie.
